Europäisches Patentamt

European Patent Office

Office européen des brevets

(19)

(11) Publication number: **0 088 570**
A1

# EUROPEAN PATENT APPLICATION

(21) Application number: **83301049.9**

(22) Date of filing: **28.02.83**

(51) Int. Cl.³: **A 61 M 1/03**, F 16 K 11/083

(30) Priority: **04.03.82 GB 8206401**

(43) Date of publication of application: **14.09.83**
**Bulletin 83/37**

(84) Designated Contracting States: **BE CH DE FR IT LI LU NL SE**

(71) Applicant: **DUNLOP LIMITED, Dunlop House Ryder Street St. James's, London SW1Y 6PX (GB)**

(72) Inventor: **Hinchliffe, John Michael, Pippins Gedges Hill Matfield, Tonbridge Kent (GB)**

(74) Representative: **Waller, Roy Ernest Sykes et al, Group Patent Department Dunlop Limited 2 Parade, Sutton Coldfield West Midlands B72 1PF (GB)**

(54) **Fluid control means.**

(57) A fluid flow control valve, suitable for use percutaneously as a dialysis valve, comprises first and second members (1, 2) movable between two relative positions, one position being for dialysis and the other for substantially normal blood circulation.

In the non-dialysis position blood flows between a first pair of inlet and outlet passages (D1, D2) in the first member (1) via a through-passage (7) in the second member (2). In the dialysis position the second member (2) diverts the flow between said inlet and outlet passages (D1, D2) to a dialysis machine, and when in said position the through-passage (7) of the second member (2) communicates with a second pair of inlet and outlet passages (B1, B2) in the first member (1) via which cleansing fluid may be circulated to cleanse said through-passage (7).

In the non-dialysis position cleansing fluid may be circulated through those passages (E1, E2) of the second member (2) which, when in the dialysis position, divert blood to the dialysis machine.

Preferably the first member (1) is adapted for location percutaneously and formed of a carbon composite or like material having good biocompatibility.

0088570

FLUID CONTROL MEANS

This invention relates to fluid flow control means and in particular, though not exclusively, to fluid flow control means for the control of extra corporeal circulation.

In instances where extra corporeal circulation is required for purposes such as dialysis it is the conventional practice to provide "shunts" in a vein and artery; a portion of the blood flow through an artery is fed through a percutaneous shunt for extra corporeal circulation and, subsequent to dialysis / treatment, is returned through another shunt to a vein. The shunts conventionally are each in the form of a length of plastics tubing having near to one end a flange portion which assists in securing the shunt relative to the patient's skin. These shunts work satisfactorily when initially installed but tend to degrade or collapse after a relatively short period of time because the neighbouring vein and artery inherently tend to become blocked in reaction to the presence of the plastics tubing. Also said shunts are prone to contamination and consequential risk of infection.

An alternative to the use of a shunt as aforedescribed is the fistula. However, this technique suffers the disadvantage that for each session of dialysis treatment it is necessary to pucture the patient's skin in order to provide connection between the fistula and the dialysis membrane.

The present invention seeks to provide a fluid flow control valve in which the aforedescribed disadvantages are at least in part overcome.

In accordance with one aspect of the present invention a fluid flow control valve comprises:

a first member having two pairs of inlet and outlet passages;

a second member movable relative to said first member such that the inlet and outlet passages of each pair are selectively interconnectable; said second member comprising a through-passage which for a selected relative position of the first and second members provides direct interconnection of one of the pairs of inlet and outlet passages of the first member; the second member further comprising a pair of inlet and outlet passages which interconnect with the pair of the inlet and outlet passages of the first member other than that pair interconnected by the said through-passage.

Preferably the first and second members are arranged to be relatively rotatable. To permit ease of installation and use of the control valve as a percutaneous device it is preferred that the first member may be formed with a retention portion, e.g. a peripherally extending flange; this facilitates location relative to the patient's skin and thus location relative to those body conduits which are connected in situ to a pair of inlet and outlet passages in the first member.

Especially in the case of a fluid flow control valve as aforedescribed and for use percutaneously as a blood access device preferably at least the first member is formed of carbon composite material, or other material which exhibits a similar degree of biocompatibility. In contrast, the second member preferably is formed of a plastics material, such as polytetrafluoroethylene, though carbon composite and like materials may be used.

Preferably the first and second members are formed with confronting frusto-conical shaped surfaces to which the respective passages in said members extend. Said surfaces are arranged to bear one against the other in a substantially fluid-tight manner so as to resist leakage of fluid from said passages.

It is further preferred that the through-passage of the second member be a single passage formed substantially wholly within the body of the material forming the second member; however, alternatively the second member may comprise a pair of passages interconnected by tubing or like means external of the main body of the second member to interconnect said passages and thus provide the required through-passage.

In use of said fluid flow control valve for dialysis the first member is implanted at a suitable site on the patient's skin and a first pair of the inlet and outlet passages are connected respectively to an artery and a vein.

In normal (non-dialysis) conditions the second member is positioned with the through-passage therein arranged to interconnect said pair of inlet and outlet passages in the first member.

When, however, dialysis is required the inlet and outlet connections of the dialysis machine are connected respectively to the inlet and outlet passages of the second member. The second member is then moved to bring the inlet and outlet passages thereof into alignment with said first inlet and outlet passages of the first member and in consequence blood is free to circulate from an artery through the control valve to the dialysis machine and return, again through the control valve, to that vein connected to the outlet passage of the first member.

With the first and second members in the relative position referred to in the preceding paragraph the through-passage in the second member aligns with the second pair of inlet and outlet passages in the first member. Said second pair of inlet and outlet passages extend from the first member at positions accessible externally of the patient, and during dialysis said inlet and outlet passages are connected to a supply of cleansing fluid which is circulated through the through-passage of the first member. Accordingly any deposits forming in the first member during normal (non-dialysis) conditions can readily be removed whilst dialysis is in progress.

One embodiment of the present invention will now be described, by way of example, with reference to the accompanying diagrammatic drawings in which:-

Figure 1 is a cross-sectional view, on the line X-X of Figure 3, of a dialysis valve in accordance with the present invention in a normal operating position;

Figure 2 is a view similar to that of Figure 1 on the line Y-Y of Figure 3;

Figure 3 is a plan view of the dialysis valve shown in Figures 1 and 2;

Figures 4 to 6 correspond to Figures 1 to 3 but show the valve positioned for dialysis, and

Figures 5 and 6 are schematic perspective type views showing the valve of Figures 1 to 4, part cut away, in the normal and dialysis positions respectively.

A fluid flow control valve for use as a percutaneous blood access device for dialysis purposes comprises a base portion 1 which serves as the aforedescribed first member and a cone portion 2 which serves as the aforedescribed second member.

The base portion 1 is formed of carbon composite material and has a peripherally extending flange portion 4 formed with small apertures (not shown) to facilitate suturing of the valve to the patient. The base portion may additionally comprise, if desired, a fabric sewing ring (not shown) to further facilitate suturing.

The base portion has a lower surface 5 which is generally flat and arranged to face inwards when

0088570

implanted. The base portion has a frusto-conical shape surface 10 which faces in a direction away from the surface 5. Said frusto-conical shape surface has four orifices B1, B2, D1 and D2 arranged therein at $90^{\circ}$ intervals around a common circle. Orifices D1 and D2 connect through-passages in the body of the base portion 1 to small tubes 6 extending from the surface 5 at positions A1 and A2 for connection to a fistula. The other two orifices in the frusto-conical surface, orifices B1 and B2, connect through-passages in the body of the base portion 1 to two connections C1, C2 provided between the flange portion 4 and frusto-conical surface so as to be accessible externally of the patient.

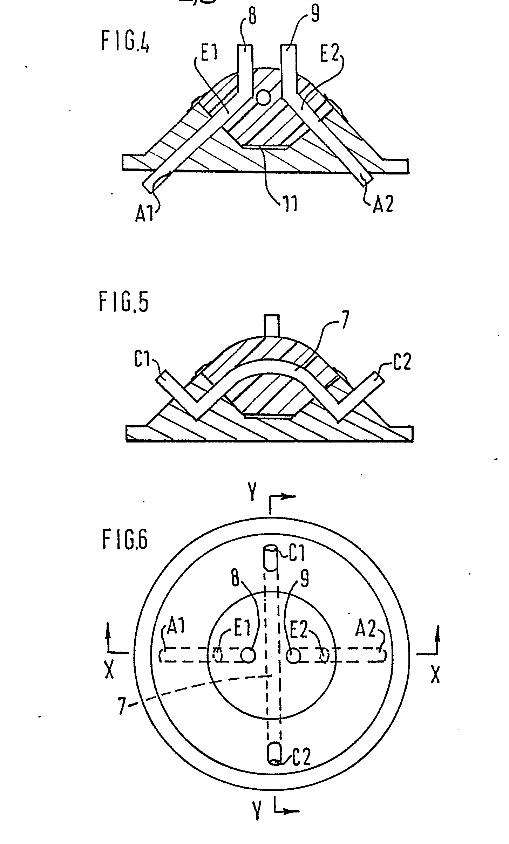

The cone portion 2 is formed of polytetrafluoroethylene and has a frusto-conical shaped surface portion which conforms closely to the similar shaped surface region of the base portion 1. The two frusto-conical shaped surfaces are maintained in close, fluid sealing contact, by means of an annular spring clip 3 (not shown in Figures 5 and 6), secured to the base portion 1 and arranged to bear against an outer surface of the cone portion 2.

The cone portion 2 has formed in the frusto-conical surface thereof four orifices arranged at $90^{\circ}$ intervals around a common centre and alignable with the orifices (B1, B2, D1 and D2) provided in the frusto-conical shape surface of the base portion 1. One pair of the orifices in the frusto-conical shape surface of the cone portion 2, a diametrically opposed pair, are interconnected by a passage 7 which extends through the body of the material forming the cone portion, whilst the other pair of diametrically opposed orifices, E1 and E2, interconnect, through small passages formed in the cone portion, to a pair of connections 8,9 respectively at an outer surface of the cone portion.

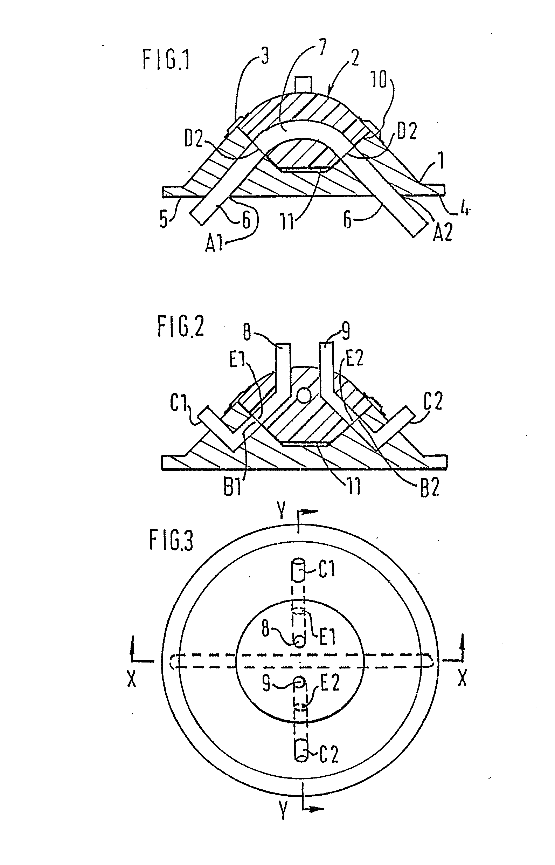

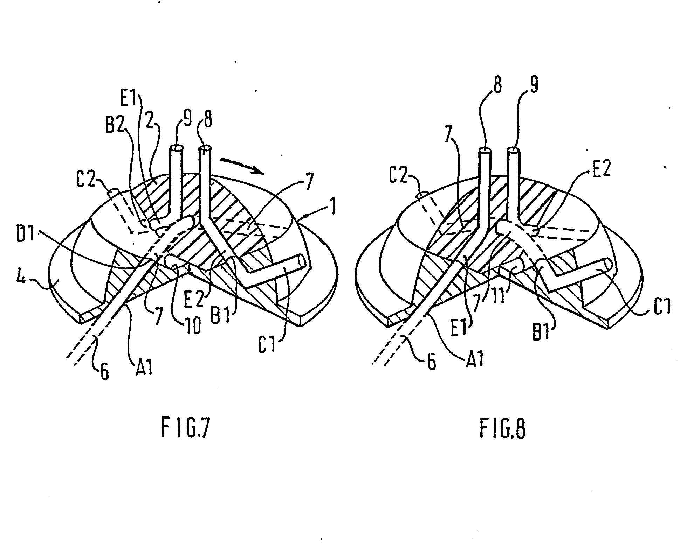

In normal use of the aforedescribed dialysis valve the position of the cone portion relative to the base portion is as shown in Figures 1 to 3. In this position the connections A1, A2 to the fistula are interconnected by the through-passage 7 in the cone portion so that blood can flow directly therethrough. In this position the four external connections C1,C2, 8 and 9 are covered with protective caps which guard against damage or contamination. When dialysis requires to be performed the cap(s) covering the external connections C1, C2, 8, 9 is (are) removed and sterilising anti-coagulant solution is then circulated between C1 and 8, and also between C2 and 9 respectively. Connections E1, E2 are then linked to the input and output respectively of a dialysis machine and the connections C1, C2 are connected to means for the supply of flushing fluid therethrough. The cone portion 2 is then rotated through 90$^{\mathrm{o}}$ relative to the base portion 1 (in the direction of the arrow of Figure 7) to bring orifice E1 into alignment with that orifice in the frusto-conical shape surface portion of the base member 1 associated with connection A1. In consequence blood flowing from the artery through tube 6 at connection A1 is led through the dialysis machine and returned to the patient through connection A2. Simultaneously, flushing fluid is passed between the external connection C1, C2 of the base portion thereby to cleanse the through-passage 7 in the cone portion 2.

When dialysis is completed the cone portion is rotated in a reverse direction through 90$^{\mathrm{o}}$ to bring the through-passage 7 (full of anti-coagulant fluid) back to its original position at which it interconnects with the fistula. The external connections C1, C2, 8, 9 are then connected to means for flushing a sterilising fluid between C1 and 8, and between C2 and 9 subsequent to which said connections are capped.

If considered necessary, in order to provide additional safeguard against the possibility of air

7.                                0088570

being introduced into the patient's bloodstream during relative movement of the base and cone portions, means may be provided for maintaining a small central space 11 between the base and cone portions under a low positive pressure by the presence therein of a suitable solution.

For constructions of the dialysis valve which are required to operate frequently or for a long period of time the frusto-conical shape surface of at least the base portion which is formed primarily of carbon composite material may be lined with a thin layer of wear resistant material such as stainless steel. A fluid sealing element may be provided between the base and cone portions to further assist in obtaining a fluid seal and excluding ingress of air.

8.  0088570

CLAIMS:

1.    A fluid flow control valve characterised in that it comprises:-

a first member (1) having two pairs of inlet and outlet passages (B1, B2, D1, D1);

a second member (2) movable relative to said first member such that the inlet and outlet passages of each pair are selectively interconnectable;

said second member comprising a through-passage (7) which for a selected relative position of the first and second members provides direct interconnection of one of the pairs of inlet and outlet passages of the first member;

the second member further comprising a pair of inlet and outlet passages (E1, E2) which interconnect with the pair of the inlet and outlet passages of the first member other than that pair interconnected by the said through-passage.

2.    A fluid flow control valve according to claim 1 characterised in that said first and second members are relatively rotatable.

3.    A fluid flow control valve according to claim 1 or claim 2 characterised in that said first and second members are formed with confronting fursto-conical shaped surfaces (10) to which the respective passages in said members extend.

4.    A fluid flow control valve according to claim 3 characterised in that said frusto-conical shaped surfaces (10) bear against one another in a substantially fluid-tight manner.

5.    A fluid flow control valve according to any one of the preceding claims characterised in that said through-passage (7) of the second member is formed substantially wholly within a body of material forming said second member.

6.    A fluid flow control valve according to any one of the preceding claims characterised in that at least said first member (1) is formed of carbon composite material, or other material which exhibits a similar degree of biocompatibility.

7.     A fluid flow control valve according to any one of the preceding claims characterised in that the confronting surfaces to which the passages of the first and second members extend are urged to bear one against the other under the action of spring means (3).

8.     A fluid flow control valve according to any one of the preceding claims characterised in that a space (11) is provided between said first and second members, in communication with the confronting surfaces of said first and second members, and is maintained at a positive pressure.

9.     A percutaneous device for extra-corporeal circulation characterised in that it comprises a fluid flow control valve according to any one of the preceding claims.

10.     A percutaneous device according to claim 9 characterised in that confronting surfaces to which the passages of the first and second members extend are subject to pressurised anti-coagulant physiological fluid.

0088570

FIG.1

3  7  2  10

D2

D2

1

5  6  11  6  4

A1  A2

FIG.2

8  9

E1  E2

C1  C2

B1  11  B2

Y

FIG.3

C1

8  E1

X  X

9  E2

C2

Y

FIG.4

FIG.5

FIG.6

0088570

FIG.8

FIG.7

European Patent
Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | US-A-3 907 688 (CLOSE) <br> * Whole document, in particular figures 2-5 * | 1-7,9 | A 61 M 1/03 <br> F 16 K 11/083 |
| Y | US-A-1 593 300 (HARWOOD) <br> * Figures 2,4, reference signs 16,18,20,22,50; page 1, line 110 - page 2, line 5; figure 10 * | 1-7,9 | |
| A | | 8 | |
| Y | GB-A-2 073 027 (RENAL SYSTEMS) <br> * Whole document, in particular page 2, lines 67-92 * | 6,9 | |
| A | US-A-4 108 173 (GENERAL ATOMIC) <br> * Whole document, in particular figure 7; column 1, lines 4-12 * | 10 | TECHNICAL FIELDS SEARCHED (Int. Cl. ³) |
| A | WO-A-7 901 121 (FRIEDMAN) <br> * Figure 1 * | 10 | A 61 M <br> F 16 K |
| A | NL-A-6 914 544 (COMPAGNIE FRANCAISE DES PETROLES) <br> * Figure 5 * | 1 | |
| A | WO-A-8 103 424 (BENTLEY) <br><br> * Figures 1,6 * <br><br> --- -/- | | |

The present search report has been drawn up for all claims

| Place of search <br> THE HAGUE | Date of completion of the search <br> 16-05-1983 | Examiner <br> PETZUCH M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO Form 1503. 03.82

# European Patent Office

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-3 626 938 (VERSACI)<br><br>* Abstract; figure 5 *<br><br>----- | | |

TECHNICAL FIELDS SEARCHED (Int. Cl. 3)

The present search report has been drawn up for all claims

| Place of search<br>THE HAGUE | Date of completion of the search<br>16-05-1983 | Examiner<br>PETZUCH M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

        
& : member of the same patent family, corresponding document

EPO Form 1503. 03.82